# FASCICULE DE BREVET EUROPEEN

(11) **EP 1 429 719 B1**
(45) Date de publication et mention de la délivrance du brevet: **01.03.2006**
(21) Numéro de dépôt: 02783184.1
(22) Date de dépôt: 20.09.2002
(51) Int. Cl.: A61K 8/368, A61K 8/42, A61Q 19/00

(54) **COMPOSITION DERMOCOSMETOLOGIQUE A VISEE ANTI-INFLAMMATOIRE, EN PARTICULIER POUR LE TRAITEMENT DE L'ACNE ET DE LA DERMITE SEBORRHE QUE**
ENTZÜNDUNGSHEMMENDE DERMATOKOSMETISCHE ZUSAMMENSETZUNG ZUR BEHANDLUNG VON AKNE UND SEBORRHOISCHER DERMATITIS
ANTI-INFLAMMATORY DERMOCOSMETOLOGIC COMPOSITION IN PARTICULAR FOR TREATING ACNE AND SEBORRHEIC DERMATITIS

(30) Priorité: 24.09.2001 FR 0112259; 18.02.2002 FR 0202001
(43) Date de publication de la demande: 23.06.2004
(73) Titulaire: Pierre Fabre Dermo-Cosmetique, 92100 Boulogne-Billancourt (FR)
(72) Inventeur: FORT-LACOSTE, Lydie, F-31860 Labarthe sur Leze (FR); ARIES, Marie-Françoise, F-31750 Escalquens (FR); DUSSERT, Anne-Sophie, F-31500 Toulouse (FR)
(74) Mandataire: Ahner, Francis
(86) Numéro de dépôt international: PCT/FR2002/003220
(87) Numéro de publication internationale: WO 2003/026604

(56) Documents cités:
- WO-A-93/07856
- US-A- 4 021 572
- US-A- 5 482 710
- US-A- 5 609 875
- DATABASE WPI Section Ch, Week 200005 Derwent Publications Ltd., London, GB; Class B05, AN 2000-055821 XP002201787 & ES 2 137 125 A (LAB VINYALS SA), 1 décembre 1999 (1999-12-01) cité dans la demande
- DATABASE WPI Section Ch, Week 199731 Derwent Publications Ltd., London, GB; Class B04, AN 1997-333297 XP002201788 & CN 1 106 258 A (AIR FORCE GEN HOSPITAL), 9 août 1995 (1995-08-09)
- DATABASE WPI Section Ch, Week 200170 Derwent Publications Ltd., London, GB; Class B04, AN 2001-609095 XP002201789 & JP 2001 181174 A (EBA KASHOHIN KK), 3 juillet 2001 (2001-07-03)
- PATENT ABSTRACTS OF JAPAN vol. 018, no. 558 (C-1264), 25 octobre 1994 (1994-10-25) & JP 06 199648 A (KAO CORP), 19 juillet 1994 (1994-07-19)
- PATENT ABSTRACTS OF JAPAN vol. 1999, no. 12, 29 octobre 1999 (1999-10-29) & JP 11 199501 A (NONOGAWA SHOJI KK), 27 juillet 1999 (1999-07-27)
- PATENT ABSTRACTS OF JAPAN vol. 1996, no. 01, 31 janvier 1996 (1996-01-31) & JP 07 242529 A (KANEBO LTD), 19 septembre 1995 (1995-09-19)

## Description

La présente invention concerne une composition dermocosmétologique à visée anti-inflammatoire et en particulier destinée au traitement de l'acné et de la dermite séborrhéïque.

Lorsque l'on s'intéresse à la physio-pathologie de l'acné, on constate que plusieurs facteurs participent au développement de l'acné qui réalise une folliculite comédonienne (« Physiopathologie de l'acné », S. Hilab,BEDC, 1998, 6,5 , 155-162).

Au sein du follicule pilo-sébacé, il existe une interaction entre ces facteurs qui sont au nombre de 4 :
* La séborrhée, qui est sous dépendance des androgènes, notamment de la testostérone et de sa transformation en dihydrotestostérone par la 5 alpha réductase ; c'est l'isoenzyme de type 1 qui est impliqué dans l'acné, avec des taux d'activité 2 à 20 fois plus élevés que la normale.
* L'hyperkératinisation du follicule, avec hyperplasie de la couche granuleuse et hypertrophie de la couche cornée ; plusieurs facteurs sont incriminés, notamment des modifications dans la composition du sébum (élévation des taux de squalène, déficit en acide linoléïque, etc...).
* La colonisation par des micro-organismes (dont Propionibacterium acnes), qui est une conséquence de la comédogénèse.
* L'inflammation, où P. acnes est largement impliqué : il possède notamment une lipase capable d'hydrolyser les triglycérides du sébum en acides gras irritants. Par la production de facteurs chimiotactiques et le déclenchement de réactions immunologiques, il propage ainsi l'inflammation avec rupture de la paroi folliculaire. Enfin, l'injection de P. acnes dans les kystes non inflammatoires entraîne une inflammation sévère.

Bien que l'isotrétinoïne ait transformé le pronostic des grandes acnés nodulokystiques, et les anti-androgènes celui des acnés féminines sévères avec hyper-androgénie, le traitement local reste cependant indiqué dans la grande majorité des acnés, parfois accompagné d'une brève antibiothérapie par voie générale.

Les principales molécules du traitement local (« Quoi de neuf dans le traitement de l'acné ? » F. Daniel, B. Dreno, M. Chivot, N. Auffret , Réalités thérapeutiques en Dermato-Vénérologie , n°57, Avril 96. « Actualités des traitements de l'acné. Première partie : traitements locaux » G. Tillès, BEDC , 1994, 2,7,312-321) sont représentées par :

### Les rétinoïdes :

L'adapalène fait partie des rétinoïdes de 2ème génération. La molécule a une affinité assez spécifique pour le récepteur de l'acide rétinoïque RAR-γ (exprimé dans le kératinocyte en phase terminale) et ne se lie pas à la protéine porteuse CRABP, ce qui améliorerait sa biodisponibilité intra-cellulaire. En application locale, elle est aussi efficace que les rétinoïdes de 1 ère génération sur les lésions rétentionnelles ; elle semble plus précocement efficace sur les lésions inflammatoires que les rétinoïdes de référence (trétinoïne et isotrétinoïne).

Ces molécules restent contre-indiquées chez la femme enceinte, de par leur potentiel tératogène.

### Le peroxyde de benzoyle :

Il exerce une activité bactéricide sur P. acnes ; il est peu comédolytique. Il peut entraîner des irritations et présente aussi un risque de photo-sensibilisation. On l'associe volontiers à d'autres molécules : association à l'érythromycine pour empêcher la sélection de nouvelles souches résistantes ; synergie d'action avec la trétindïne, etc.

### L'acide azélaïque :

Il est utilisé pour ses propriétés anti-microbiennes. Sa tolérance est meilleure que celle de la trétinoïne ou du peroxyde de benzoyle, mais son intérêt dans l'acné reste faible.

### Le nicotinamide :

Il est utilisé pour son activité anti-inflammatoire. Cet effet semble être la résultante d'une action sur différents facteurs de l'inflammation (inhibition du chimiotactisme des neutrophiles, inhibition du relargage d'histamine à partir des mastocytes,...). Il n'a aucune action sur la flore bactériologique de l'acné.

### Les acides alpha-hydroxylés :

Leur activité est fonction de la concentration, mais aussi de la longueur de la chaîne carbonée et du pH. Ils sont employés dans le traitement de l'acné. Ils diminuent l'hyperkératinisation folliculaire, grâce à leur action de réduction de la cohésion coméocytaire au niveau de la partie supérieure des follicules pileux. Des associations à d'autres traitements sont rencontrées (complémentarité d'action avec le peroxyde de benzoyle,...).

Les traitements hormonaux locaux ont une efficacité clinique très décevante.

Quant à la question de l'efficacité respective de l'antibiothérapie locale et de l'antibiothérapie générale, elle est souvent controversée.

L'enoxolone, encore appelé acide 18β-glycyrrhétinique, est un principe actif anti-inflammatoire topique ; il est issu de racines de réglisse (Glycyrrhiza glabra). Sa structure est la suivante :

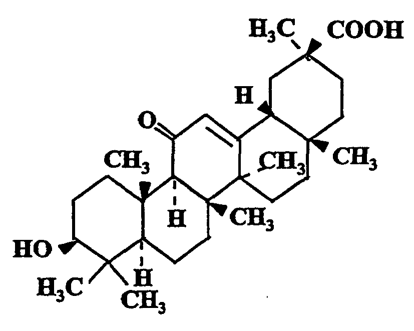

Il s'agit de la partie aglycone de la glycyrrhizine, qui est un saponoside triterpénique.

Ses propriétés anti-inflammatoires ont donné lieu à de nombreuses publications, notamment dans le traitement de dermatoses (Colin-Jones E., Somers G.F., A non steroidal anti-inflammatory agent in Dermatology. The Medical Press, 1957, 238, 6173, 206). Son activité, de type cortisone-like, ne s'accompagne pas d'effets indésirables du fait de la structure non stéroïdienne de la molécule.

Parmi ses dérivés, le stéaryl glycyrrhétinate, obtenu par estérification de l'acide β-glycyrrhétinique avec l'alcool stéarylique, présente un caractère lipophile qui améliore les performances cutanées de la molécule.

Un autre dérivé, le glycyrrhétinate de zinc, est également à prendre en considération, notamment dans le cadre du traitement topique de l'acné (brevet Laboratorios Dr VINYALS n° 2137125).

En effet, le zinc tient une place importante dans la biologie des métallo-enzymes, la synthèse protéique, les phénomènes immunologiques et le métabolisme des hormones.

Il exerce notamment, au niveau des androgènes, une action inhibitrice au niveau de la 5α-réductase. La peau et les phanères (zinc et peau - Dermatologie - 2ème édition -2-1980) renferment 20 % de la teneur globale en zinc ; il y joue notamment un rôle dans les processus de kératinisation.

L'efficacité du zinc dans l'acné inflammatoire remonte à 1970 ; différents sels sont utilisés (gluconate, sulfate, etc.). Il joue également un rôle dans le traitement de la dermite séborrhéïque.

Son utilisation sous forme de glycyrrhétinate est donc particulièrement pertinente pour les traitements de l'acné, et de la dermite séborrhéïque.

Parmi les humectants utilisés en dermo-cosmétologie, les lactamides présentent des propriétés intéressantes, notamment le lactamide MEA.

Il s'agit d'un monoéthanolamide de l'acide lactique, dont la structure chimique est la suivante :

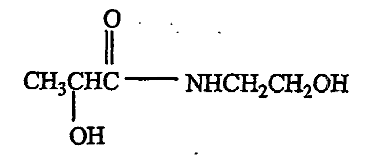

Ce produit est fabriqué à partir d'acide lactique et de monoéthanolamine ; on obtient un produit de pureté de 97 % minimum.

C'est un agent antistatique, substantif sur la peau et les cheveux (New substantive humectants - J. Soc. Cosmet. Chem., 38 (5) 1987 - 357).

Il a été proposé dans l'art antérieur pour le traitement de l'acné (Brevet Van Scott - US 4 021 572).

Conformément à la présente invention, la composition dermocosmétologique comprend, à titre de principes actifs, une association synergique de glycyrrhétinate de zinc et/ou de glycyrrhétinate de stéaryl et/ou de glycyrrhétinate d'ammonium avec au moins un lactamide. Ce lactamide est de préférence constitué par le monoéthanolamide de l'acide lactique.

La quantité de glycyrrhétinate dans les formulations est avantageusement comprise entre 0,01 % et 5% en poids et celle de lactamide entre 0,5 % et 10% en poids.

La quantité préférée de glycyrrhétinate est de 1% en poids et celle de lactamide de 2% en poids.

La présente invention s'étend également à l'utilisation d'une association synergique de glycyrrhétinate de zinc et/ou de glycyrrhétinate de stéaryl et/ou de glycyrrhétinate d'ammonium avec au moins un lactamide et en particulier avec le monoéthanolamide de l'acide lactique pour la fabrication d'un médicament anti-inflammatoire, notamment pour le traitement de l'acné et de la dermite séborrhéïque.

La composition dermocosmétologique selon l'invention comprend, en outre, des excipients usuels dennatologiquement compatibles, tel qu'illustré ultérieurement dans la description par un certain nombre d'exemples de formulations particulières.

Conformément à la présente invention, il a été mis en évidence une synergie d'activité anti-inflammatoire du lactamide et du glycyrrhétinate de zinc et/ou du glycyrrhétinate de stéaryl et/ou de glycyrrhétinate d'ammonium. Cette synergie a été objectivée à la suite d'une évaluation sur la production de prostaglandine 6KF1α par le kératynocyte humain.

Le kératinocyte est la cellule la plus représentée au niveau de l'épiderme. En réponse à de nombreux facteurs extra-cellulaires présents dans son environnement, il libère divers médiateurs biologiquement actifs, notamment les prostaglandines et les leucotriènes qui jouent un rôle important dans l'initiation et la modulation des réactions inflammatoires cutanées et qui interviennent également dans la régulation de la réponse immune.

Le kératinocyte se révèle être un bon modèle d'étude en pharmacologie cutanée ; ce modèle cellulaire permet de déterminer, in vitro, les capacités de diverses molécules à moduler la production de ces médiateurs issus du métabolisme de l'acide arachidonique.

Dans cette étude, on s'est intéressé en particulier à une prostaglandine, la PG6KF1α, métabolite stable de la prostarycline, qui est un des métabolites majeurs produits par le kératinocyte stimulé, et représentatif de la modulation de la production des métabolites de l'acide arachidonique issus de la voie de la cyclo-oxygénase.

Sur ce modèle, on a évalué l'activité du glycyrrhétinate de zinc en association avec le lactamide sur la production de PG6KF1α induite chez le kératinocyte par un stimulant de la cascade de l'acide arachidonique, le ionophore calcique A23187.

On a réalisé ensuite la même étude en évaluant l'activité du stéaryl glycyrrhétinate en association avec le lactamide.

Le protocole mis en oeuvre est le suivant :

La suspension de kératinocytes humains HaCaT dans le RPMI 10% SVF est distribuée dans les plaques 6 puits (1x106 cellules/2ml/puits) et incubée pendant 16 heures à 37°C dans une atmosphère à 5% CO₂. Les kératinocytes sont alors rincés avec du PBS pour éliminer les cellules non-adhérentes puis exposés aux produits à tester inclus dans du RPMI sans SVF (qui pourrait interférer dans le dosage).

Le lactamide MEA est directement solubilisé dans le RPMI sans SVF à une concentration de 1 0mg/ml.

Le glycyrrhétinate de zinc est préparé dans l'éthanol absolu à 100 mg/ml puis repris dans le RPMI sans SVF à 10 mg/ml.

Le stéaryl glycyrrhétinate est préparé dans le DMSO à 1000 µg/µl puis repris dans le RPMI sans SVF à 10mg/ml.

Le glycyrrhétinate d'ammonium est préparé dans l'éthanol absolu à 100 mg/ml puis repris dans le RPMI sans SVF à 25 mg/ml.

Pour chaque lot, 3 puits sont réalisés. Les cellules sont incubées pendant 1 heure avec les produits à tester puis le ionophore calcique A23187, est ajouté pendant 5 heures à la concentration 5µM.

Les milieux de culture de chacun des puits sont ensuite récupérés, centrifugés à 3000 tr/mn et conservés à -80°C.

La production de prostaglandine 6KF1α pour chacun des essais est mesurée par Kit Elisa EUROMEDEX.

Les lots suivants ont été réalisés :
- Lot témoin
- Lot témoin stimulé A23187 5µM
- Lactamide MEA 500µg/ml + A23187 5µM
- Glycyrrhétinate de zinc 10 µg/ml + A23187 5µM
- Lactamide MEA 500µg/ml/Glycyrrhétinate de zinc 10µg/ml + A23187 5µM
- Stéaryl glycyrrhétinate 50µg/ml + A23187 5µM
- Lactamide MEA 500µg/ml/Stéaryl glycyrrhétinate 50µg/ml + A23187 5µM
- Glycyrrhétinate d'ammonium 25 µg/ml + A23187 5µM
- Lactamide MEA 500 µg/ml/Glycyrrhétinate d'ammonium 25 µg/ml + A23187 5µM.

Les résultats sont exprimés en picogrammes de prostaglandine 6KF1α pour 1x10⁶ kératinocytes et par ml de culture.

L'analyse statistique des résultats est réalisée par une analyse de variance et par le test de Dunnett.

Les résultats obtenus par cette étude in vitro de la modulation de la libération de la prostaglandine 6KF1α par les kératinocytes stimulés montrent que :
- le glycyrrhétinate de zinc (10 µg/ml), le stéaryl glycyrrhétinate (50µg/ml) ainsi que le glycyrrhétinate d'ammonium (25 µg/ml) présentent une activité anti-inflammatoire significative de - 32 %, -31 % et - 30 % respectivement ;
- l'association de glycyrrhétinate de zinc (10µg/ml) avec le lactamide MEA (500 µg/ml) montre une activité significativement augmentée (- 49% d'inhibition) et significativement différente du glycyrrhétinate de zinc (10 µg/ml) seul.

Il en est de même pour l'association du stéaryl glycyrrhétinate 50µg/ml ou du glycyrrhétinate d'ammonium 25 µg/ml avec le lactamide MEA 500µg/ml qui montrent chacun une activité significativement augmentée (respectivement - 42 % et - 40 % d'inhibition) et significativement différente du glycyrrhétinate seul.

En présence de lactamide, l'activité anti-inflammatoire du glycyrrhétinate de zinc, du stéaryl glycyrrhétinate ainsi que du glycyrrhétinate d'ammonium est significativement potentialisée.

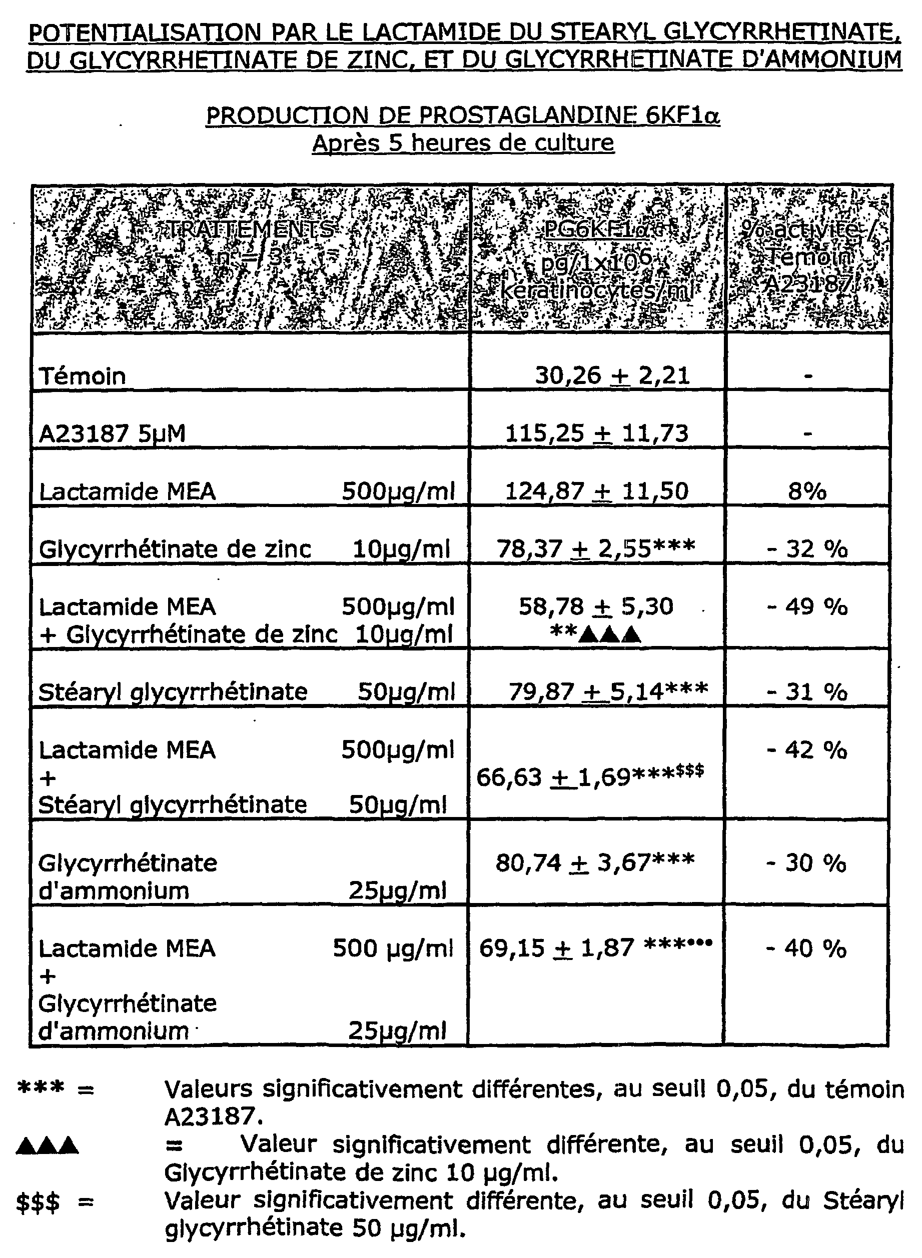

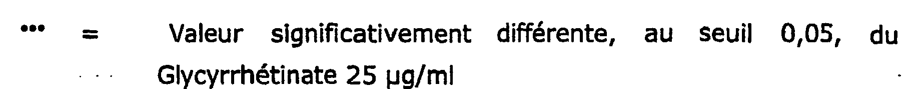

De manière à illustrer les compositions dermocosmétologiques selon l'invention, on indiquera ci-après un certain nombre d'exemples non limitatifs de formulations reprenant une telle association synergique objet de la présente invention.

**Crème H/E**

| **Ingrédients** | **Quantité (g)** |
|---|---|
| Cétéaryl alcool et Cétéareth 33 | 1-10 |
| Glycéryl monostéarate | 1-5 |
| Cétyl alcool | 0,5-1 |
| Cétyl esters | 0,5-3 |
| Caprylic/Capric Triglyceride | 0-10 |
| Dicaprylyl éther | 0-10 |
| Cyclométhicone | 0-5 |
| Acide glycolique | 0,5-10 |
| NaOH | qs pH |
| Acide salicylique | 0,1 |
| Polyméthyl méthacrylate | 0,5-2 |
| Gomme xanthane | 0,1 |
| Glycyrrhétinate de zinc ou Stearyl glycyrrhétinate | 0,01-5 |
| Lactamide MEA | 0,5-10 |
| Parfum | Qs |
| Eau déminéralisée | qsp 100g |

**Emulsion fluide H/E**

| **Ingrédients** | **Quantité (g)** |
|---|---|
| Cétéaryl alcool et Cétéareth 33 | 1-10 |
| Glycéryl monostéarate | 1-5 |
| Hydroxyoctacosanyl Hydroxystéarate | 0,5-5 |
| Dicaprylyl éther | 0-10 |
| Cyclométhicone | 0-20 |
| Butane-diol 1-3 | 0-10 |
| PEG 1500 | 0,5-10 |
| Alcool 96° | 0-15 |
| Eau d'hamamélis | 0-10 |
| Cetrimonium Bromide | 0-0,5 |
| Acide glycolique | 0,5-10 |
| NaOH | qs pH |
| Acide salicylique | 0-2 |
| Glycyrrhétinate de zinc ou Stéaryl glycyrrhétinate | 0,01-5 |
| Lactamide MEA | 0,5-10 |
| Titanium dioxide | 0-1 |
| Parfum | qs |
| Colorant | qs |
| Eau déminéralisée | qsp 100g |

**Gel crème H/E**

| **Ingrédients** | **Quantité (g)** |
|---|---|
| Sepigel 305 | 2-10 |
| Cyclométhicone | 0-10 |
| Propylène glycol | 0-10 |
| Polyméthyl méthacrylate | 0-2 |
| Glycyrrhétinate de zinc ou Stearyl glycyrrhétinate | 0,01-5 |
| Lactamide MEA | 0,5-10 |
| Parfum | qs |
| Conservateurs | qs |
| Eau déminéralisée | qsp 100g |

**Formulation H/E avec charges minérales**

| **Ingrédients** | **Quantité (g)** |
|---|---|
| Glycéryl monostéarate et PEG 100 | 2-15 |
| Cétéareth-20 | 0-5 |
| Kaolin | 1-20 |
| Titanium dioxide | 0,5-5 |
| Hectorite | 1-5 |
| Glycérine, glycols | 1-15 |
| Polyéthylène | 0-6 |
| Glcyrrhétinate de zinc ou Stearyl glycyrrhétinate | 0,01-5 |
| Lactamide MEA | 0,5-10 |
| NaOH | qs pH |
| Conservateurs | qs |
| Parfum | qs |
| Eau déminéralisée | qsp 100g |

**Emulsion E/H**

| **Ingrédients** | **Quantité (g)** |
|---|---|
| Hostacerin WO | 1-8 |
| Paraffine liquide | 0-25 |
| Caprylic/Capric Triglyceride | 0-10 |
| Huile végétale hydrogénée | 1-8 |
| Propylène glycol | 0-10 |
| Glycyrrhétinate de zinc ou Stearyl glycyrrhétinate | 0,01-5 |
| Lactamide MEA | 0,5-10 |
| Magnésium sulfate | 0,1 |
| Conservateurs | qs |
| Parfum | qs |
| Eau déminéralisée | qsp 100g |

**Formulation anhydre (Stick, compact)**

| **Ingrédients** | **Quantité (g)** |
|---|---|
| Vaseline blanche | 0-15 |
| Paraffine liquide | 0-15 |
| Caprylic/Capric Triglyceride | 0-10 |
| Palmitate d'isopropyle | 0-15 |
| Lanoline hydrogénée | 0-20 |
| Cétyl esters | 0-10 |
| Huile de ricin hydrogénée | 0-10 |
| Cire d'abeille | 0-5 |
| Cire de carnauba | 0-2 |
| Ozokérite | 0-15 |
| Glcyrrhétinate de zinc ou Stearyl glycyrrhétinate | 0,01-5 |
| Lactamide MEA | 0,5-10 |
| Parfum | qs |
| Huile de ricin | qsp 100g |

**Lotion hydroalcoolique**

| **Ingrédients** | **Quantité (g)** |
|---|---|
| Solubilisant LRI | 0,5-10 |
| Glycols | 0-10 |
| Alcool 96° | 0-10 |
| Glcyrrhétinate de zinc ou Stearyl glycyrrhétinate | 0,01-5 |
| Lactamide MEA | 0,5-10 |
| Conservateurs | qs |
| Parfum | qs |
| Eau déminéralisée | qsp 100g |

**Nettoyant moussant (savon)**

| **Ingrédients** | **Quantité (g)** |
|---|---|
| Acide laurique | 0,5-8 |
| Acide palmitique | 0,5-8 |
| Acide stéarique | 0,5-20 |
| Acide myristique | 0,5-158 |
| Decyl glucoside | 0-7 |
| Cocamide DEA | 0-7 |
| Glycérine | 0-15 |
| PEG 600 | 0-8 |
| Glcyrrhétinate de zinc ou Stearyl glycyrrhétinate | 0,01-5 |
| Lactamide MEA | 0,5-10 |
| NaOH, KOH | qs pH 9 |
| Conservateurs | qs |
| Parfum | qs |
| Eau déminéralisée | qsp 100g |

**Nettoyant moussant (sans savon)**

| **Ingrédients** | **Quantité (g)** |
|---|---|
| Lauryl ether sulfate 70% | 0-12 |
| Cocamidopropylbétaine | 0-20 |
| Decyl glucoside | 0-10 |
| Glycérine | 0-10 |
| Glcyrrhétinate de zinc ou Stearyl glycyrrhétinate | 0,01-5 |
| Lactamide MEA | 0,5-10 |
| Acide glycolique | 2-6 |
| NaOH | qs pH |
| Conservateurs | qs |
| Parfum | qs |
| Colorant | qs |
| Eau déminéralisée | qsp 100g |

**Produit avec écrans solaires**

| **Ingrédients** | **Quantité (g)** |
|---|---|
| C12 15 alkyl benzoate | 0-10 |
| Isododecane | 0-20 |
| Cyclométhicone | 0-20 |
| Dimethicone | 0,5-10 |
| Titanium dioxide | 0,5-30 |
| Oxyde de zinc | 0,1-30 |
| Triglycérides de lait hydroxylés | 0,1-5 |
| Silice hydrogénée | 0,05-2 |
| Carbomer | 0,05-2 |
| Pemulen TR1 ® | 0,05-3 |
| Glycyrrhétinate de zinc ou Stearyl glycyrrhétinate | 0,01-5 |
| Lactamide MEA | 0,5-10 |
| Sodium hydroxyde | qs pH |
| Conservateurs | qs |
| Parfum | qs |
| Eau déminéralisée | qsp 100g |

**Produit avec filtres et écrans solaires**

| **Ingrédients** | **Quantité (g)** |
|---|---|
| C12 15 alkyl benzoate | 0-15 |
| Octyldodecyl neopentanoate | 0-15 |
| Cetyl alcohol | 0-1 |
| Octylmethoxycinnamate | 0-10 |
| Titanium dioxide | 0,5-25 |
| Oxyde de zinc | 0,1-30 |
| Cyclomethicone et Dimethiconol | 1-5 |
| Sorbitan trioléate | 0,1-5 |
| Carbomer | 0,05-1 |
| Pemulen TR1 ® | 0,01-1 |
| Hydroxypropylmethylcellulose | 0-1 |
| Glycerine | 0-10 |
| Glycyrrhétinate de zinc ou Stearyl glycyrrhétinate | 0,01-5 |
| Lactamide MEA | 0,5-10 |
| TEA | qs pH |
| Conservateurs | qs |
| Parfum | qs |
| Eau déminéralisée | qsp 100g |

**Gel hydroalcoolique**

| **Ingrédients** | **Quantité (g)** |
|---|---|
| Hydroxyéthylcellulose | 0,2-5 |
| Glycols | 0-10 |
| Eau florale (Hamamélis, Rose etc) | 0-10 |
| Alcool 96° | 0-40 |
| Glycyrrhétinate d'ammonium | 0,01-5 |
| Lactamide MEA | 0,5-10 |
| Acide salicylique | 0-4 |
| Acide glycolique | 0,5-10 |
| NaOH | qs pH |
| Amidon de maïs (ou autre poudre absorbante) | 0,1-5 |
| Conservateurs | qs |
| Parfum | qs |
| Eau déminéralisée | qsp 100 g |

## Revendications

1. Composition dermocosmétologique, **caractérisée en ce qu'**elle contient, à titre de principes actifs, une association synergique de glycyrrhétinate de zinc et/ou de glycyrrhétinate de stéaryl et/ou de glycyrrhétinate d'ammonium avec au moins un lactamide.

2. Composition selon la revendication 1, **caractérisée en ce que** le lactamide est le monoéthanolamide de l'acide lactique.

3. Composition dermocosmétologique selon l'une des revendications 1 ou 2, **caractérisée en ce que** la quantité de lactamide est comprise entre 0,5 % et 10 % en poids et que la quantité de glycyrrhétinate est comprise entre 0,01 % et 5% en poids.

4. Composition dermocosmétologique selon la revendication 3, **caractérisée en ce que** le lactamide est présent à raison de 2% en poids et le glycyrrhétinate à raison de 1% en poids.

5. Utilisation d'une association synergique de glycyrrhétinate de zinc et/ou de glycyrrhétinate de stéaryl et/ou de glycyrrhétinate d'ammonium avec au moins un lactamide et en particulier avec le monoéthanolamide de l'acide lactique pour la fabrication d'un médicament anti-inflammatoire, notamment pour le traitement de l'acné et de la dermite séborrhéïque.

6. Utilisation selon la revendication 5, **caractérisée en ce que** la quantité de lactamide est comprise entre 0,5 % et 10 % en poids et la quantité de glycyrrhétinate est comprise entre 0,01 % et 5% en poids.

7. Utilisation selon la revendication 6, **caractérisée en ce que** le lactamide est présent à raison de 2% en poids et le glyccyrrhétinate est présent à raison de 1% en poids.

## Patentansprüche

1. Dermatokosmetische Zusammensetzung, **dadurch gekennzeichnet, dass** sie als Wirkstoffe eine synergistische Kombination von Zinkglycyrrhetinat und/oder Stearylglycyrrhetinat und/oder Ammoniumglycyrrhetinat mit wenigstens einem Lactamid enthält.

2. Zusammensetzung nach Anspruch 1, **dadurch gekennzeichnet, dass** das Lactamid das Monoethanolamid von Milchsäure ist.

3. Dermatokosmetische Zusammensetzung nach einem der Ansprüche 1 oder 2, **dadurch gekennzeichnet, dass** die Lactamid-Menge zwischen 0,5 Gew.-% und 10 Gew.-% eingeschlossen liegt und dass die Glycyrrhetinat-Menge zwischen 0,01 Gew.-% und 5 Gew.-% eingeschlossen liegt.

4. Dermatokosmetische Zusammensetzung nach Anspruch 3, **dadurch gekennzeichnet, dass** das Lactamid in einer Konzentration von 2 Gew.-% und das Glycyrrhetinat in einer Konzentration von 1 Gew.-% vorhanden ist.

5. Verwendung einer synergistischen Kombination von Zinkglycyrrhetinat und/oder Stearylglycyrrhetinat und/oder Ammoniurnglycyrrhetinat mit wenigstens einem Lactamid und insbesondere mit dem Monoethanolamid von Milchsäure für die Herstellung eines entzündungshemmenden Arzneimittels, insbesondere für die Behandlung von Akne und von seborrhoischer Dermatitis.

6. Verwendung nach Anspruch 5, **dadurch gekennzeichnet, dass** die Lactamid-Menge zwischen 0,5 Gew.-% und 10 Gew.-% eingeschlossen liegt und die Glycyrrhetinat-Menge zwischen 0,01 Gew.-% und 5 Gew.-% eingeschlossen liegt.

7. Verwendung nach Anspruch 6, **dadurch gekennzeichnet, dass** das Lactamid in einer Konzentration von 2 Gew.-% und das Glycyrrhetinat in einer Konzentration von 1 Gew.-% vorhanden ist.

## Claims

1. Dermocosmetological composition, **characterized in that** it contains, as active principles, a synergistic combination of zinc glycyrrhetinate and/or of stearyl glycyrrhetinate and/or of ammonium glycyrrhetinate with at least one lactamide.

2. Composition according to Claim 1, **characterized in that** the lactamide is lactic acid monoethanolamide.

3. Dermocosmetological composition according to either of Claims 1 and 2, **characterized in that** the amount of lactamide is between 0.5% and 10% by weight and that the amount of glycyrrhetinate is between 0.01% and 5% by weight.

4. Dermocosmetological composition according to Claim 3, **characterized in that** the lactamide is present in a proportion of 2% by weight and the glycyrrhetinate in a proportion of 1% by weight.

5. Use of a synergistic combination of zinc glycyrrhetinate and/or of stearyl glycyrrhetinate and/or of ammonium glycyrrhetinate with at least one lactamide, and in particular with lactic acid monoethanolamide, for the manufacture of an anti-inflammatory medicinal product, in particular for treating acne and seborrhoeic dermatitis.

6. Use according to Claim 5, **characterized in that** the amount of lactamide is between 0.5% and 10% by weight and the amount of glycyrrhetinate is between 0.01% and 5% by weight.

7. Use according to Claim 6, **characterized in that** the lactamide is present in a proportion of 2% by weight and the glycyrrhetinate is present in a proportion of 1% by weight.
